# EUROPEAN PATENT SPECIFICATION

(11) **EP 1 903 046 B1**
(45) Date of publication and mention of the grant of the patent: **16.02.2011**
(21) Application number: 07116866.0
(22) Date of filing: 20.09.2007
(51) Int. Cl.: C07D 495/04, A61K 31/4365, A61P 7/02

(54) **New clopidogrel salt and its crystalline forms**
Neues Clopidogrel-Salz und seine kristallinen Formen
Nouveau sel de clopidogrel et ses formes cristallines

(30) Priority: 25.09.2006 PL 38067706; 29.08.2007 PL 38321507
(43) Date of publication of application: 26.03.2008
(73) Proprietor: Adamed SP. Z O.O., 05-152 Czosnów k/Warszawy (PL)
(72) Inventor: Majka, Zbigniew, 39-102, Lubzina (PL); Sawicki, Andrzej Wojciech, 05-860, Józefów (PL); Trzepalka, Andrzej, 05-480, Karczew (PL); Adamkiewicz, Malgorzata, 02-761, Warszawa (PL)
(74) Representative: Hawrylak, Jolanta

(56) References cited:
- WO-A-2004/106344
- US-A1- 2005 256 152

## Description

### FIELD OF THE INVENTION

The present invention relates to new clopidogrel salt, to its crystalline forms, to processes for preparation of the new salt and its crystalline forms, to pharmaceutical compositions comprising the new salt or its crystalline forms, and to use of the new salt or its crystalline forms in medicine and also in the manufacture of a medicament for treatment platelet aggregation related diseases.

### TECHNICAL BACKGROUND

Clopidogrel is a generic name of methyl (+)-(S)-α-(2-chlorophenyl)-6,7-dihydrothieno[3,2-c]pyridine-5(4H)-acetate of Formula A, and is a well-known platelet aggregation inhibitor. The compound, its activity and use in medicine were disclosed in the patent publication EP 0099802.

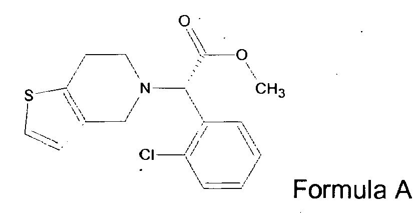

Patent publication EP-281459 discloses pharmaceutically acceptable clopidogrel salts, including hydrogen sulfate. Crystalline clopidogrel hydrogen sulfate is present in a composition of currently marketed medicinal product. The inventors of an invention described in the patent publication EP-281459 state that hydrogen sulfate has the most beneficial properties among obtained clopidogrel salts. Other salts disclosed in this publication include clopidrogel hydrochloride, hydrobromide, carboxylic acid salts: acetate, benzoate, fumarate, maleate, citrate, tartrate, gentisate, and sulfonic acid salts: taurocholate, methanesulfonate, ethanesulfonate, benzenesulfonate, lauryl sulfonate, dobesilate. It was mentioned that clopidogrel salts with organic acids are amorphous and/or hygroscopic, what makes them difficult to purify and renders their use in the manufacture of pharmaceutical compositions problematic.

Various polymorphic forms of salts known from the above publication were described, for example clopidogrel benzenesulfonate in patent publications WO 2004/106344, WO 2004/072084, clopidogrel hydrochloride in WO 2005/117866, and clopidogrel hydrobromide in WO 2005/068471.

There are also publications, which describe new clopidogrel salts. For example clopidogrel 2-propylsulfate and clopidogrel perchlorate are described in WO 2005/016931, naphtalenesulfonate and naphtalenedisulfonate in WO 2005/097804, and clopidogrel salts with alkyl-sulfuric acids in patent publication EP-1415993A. A broad list of salts is presented in WO 2004/074215, however preparation processes and properties were disclosed for clopidogrel mesylate, sulfonate, acetate, hydrochloride, hydroiodide, perchlorate and nitrate only.

Clopidogrel hydrogen sulfate used in the marketed product exhibits some disadvantages. Sulfuric acid makes this salt strongly acidic. Such acidic characteristic results in incompatibility of this salt with many additives. This influences the stability of the pharmaceutical compositions. It was observed that powder composition containing clopidogrel hydrogen sulfate for filling of gelatine capsules is aggressive, and clopidogrel hydrogen sulfate causes perforation of capsule shells. Other disadvantages are related to the process of tablet manufacture. Clopidogrel hydrogen sulfate as such as well as in a powder for making tablets is strongly aggressive. This feature is of great importance in preparation and processing, since workers need to be equipped with additional protection means. Clopidogrel hydrogen sulfate also causes corrosion of metallic punches of tableting machine.

There is a demand in the art for a new clopidogrel salt, which would be not so aggressive and would be more stable and more suitable for technological processing, than hydrogen sulfate which is used in the marketed pharmaceutical formulations.

The inventors of present invention have found such a new clopidogrel salt with a pharmaceutically acceptable acid, in particular 5-sulfosalicylic acid. This salt is stable, non-hygroscopic and easily undergoes pharmaceutical processing. The new clopidogrel salt as described herein is devoided of aggressive properties and does not cause machine elements corrosion.

### SUMMARY OF THE INVENTION

In the first aspect, the present invention provides a new clopidogrel salt with 5-sulfosalicylic acid, the salt having given below structure of Formula I.

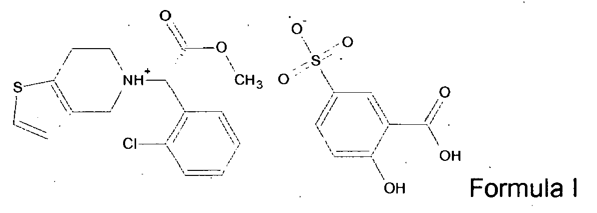

In the second aspect, the present invention provides clopidogrel 5-sulfosalicylate in two crystalline forms, Form I and Form II, which differ in respect of physical properties and have different X-ray powder diffraction patterns (XRPD) and FT-IR spectra.

In another aspect, the present invention provides processes for preparation of said clopidogrel 5-sulfosalicylate and its crystalline forms.

The present invention further relates to a pharmaceutical composition comprising clopidogrel salt of the present invention or its crystalline forms.

The present invention further relates to use of clopidogrel 5-sulfosalicylate as herein described in medicine and also in the manufacture of a medicament for treatment of platelet aggregation related diseases.

### BRIEF DESCRIPTION OF THE FIGURES

Figure 1 presents FT IR spectrum of crystalline Form I of clopidogrel 5-sulfosalicylate.
Figure 2 presents X-ray powder diffraction pattern of crystalline Form I of clopidogrel 5-sulfosalicylate.
Figure 3 presents FT IR spectrum of crystalline Form II of clopidogrel 5-sulfosalicylate.
Figure 4 presents X-ray powder diffraction pattern of crystalline Form II of clopidogrel 5-sulfosalicylate.

### DETAILED DESCRIPTION OF THE INVENTION

The new salt, clopidogrel 5-sulfosalicylate, is more stable and less susceptible to degradation caused by hydrolysis of the ester group than clopidogrel hydrogen sulfate, which is generally used in medicinal products. The susceptibility to hydrolysis, for example under the influence of moisture, increases with the strength of acid. Sulfuric acid as stronger acid (pKₐ₁= -3) than 5-sulfosalicylic acid (pKₐ₁= -0,8) increases the susceptibility of the ester group of clopidogrel to acidic hydrolysis.

Clopidogrel 5-sulfosalicylate has very beneficial properties. It is characterized by high storage stability, among others non-hygroscopicity, lack of light sensitivity and solubility profile which is suitable for oral compositions. It is known that optical isomer of clopidogrel, methyl (-)-(R)-α-(2-chlorophenyl)-6,7-dihydrothieno[3,2-c]pyridine-5(4H)-acetate, is toxic on central nervous system (see the toxicity in the patent publication EP 281459). Therefore stability of the proper enantiomer of this compound is of a great importance.

Process for preparation of clopidogrel salt with 5-sulfosalicylic acid, consists in that clopidogrel base is reacted with 5-sulfosalicylic acid.

Preferably clopidogrel is used in the form of a solution in an organic solvent, most preferably in methylene chloride or chloroform, and crystalline salt is obtained. Clopidogrel 5-sulfosalicylate is sparingly soluble in those solvents and easily crystallizes with almost 100% yield. The present inventors have found that other solvents: alcohols (methanol, ethanol, isopropanol), esters (ethyl acetate), hydrocarbons (toluene), ketones (acetone, methyl isobutyl ketone), ethers (diethyl ether, methyl tert-butyl ether) cause formation of said salt in oily form. Even after solvent evaporation the remained salt is in the form of viscous oil. But adding methylene chloride or chloroform to such liquid residue after evaporation of another solvent causes crystallization of the oil and formation of clopidogrel 5-sulfosalicylate in a crystalline Form I.

Preferably 5-sulfosalicylic acid is used as crystalline dihydrate which is easily accessible.

Clopidogrel in the form of a base, that is essential in the process of the invention, may be prepared with methods known from the state of the art, for example by releasing from diastereomeric salt with camphorsulfonic acid as disclosed in patent publication EP-281459.

The claimed process may be carried out in rather wide temperature range, from ambient temperature (i.e. about 20°C) to the solvent boiling temperature.

Salt precipitation from the reaction mixture may be spontaneous or induced by cooling. Salt precipitated from the solution may be isolated with conventional methods, preferably by filtration. The salt in the oil form may be converted into crystalline Form I by adding methylene chloride or chloroform to the oily residue obtained after evaporation of a different solvent, and stirring till crystalline salt /Form I/ is precipitated.

Precipitated product /salt/ may be dried, both in a common dryer or vacuum dryer, in a wide range of temperatures: from ambient temperature to 100°C.

Clopidogrel salts may be prepared in so called double replacement reaction. Claimed salt may be prepared in a process wherein the acid addition salt of clopidogrel is reacted with alkaline metal or alkaline earth metal salt of 5-sulfosalicylic acid. Suitable acid addition salts of clopidogrel include, for example, hydrogen sulfate and hydrochloride, and suitable 5-sulfosalicylic acid salt include sodium, potassium, lithium, calcium and magnesium salt. The process may be carried out in a solvent, for example selected from a group of lower alcohols, at the temperature between room temperature and the solvent boiling temperature. After the reaction is completed the mixture is filtered off and filtrate is concentrated under reduced pressure. The residue is dissolved in a solvent, for example in acetone. Inorganic salts also precipitate from the solution as a by-product. After filtration of those salts the filtrate is again concentrated to obtain clopidogrel 5-sulfosalicylate in an oil form. To obtain crystalline Form I of clopidogrel 5-sulfosalicylate, such obtained oil is mixed with a solvent from which the requested salt easily crystallizes, such as methylene chloride or chloroform. This method is not a convenient method of obtaining the new salt, since in comparison to the claimed reaction of clopidogrel with 5-sulfosalicylic acid it requires many additional operations.

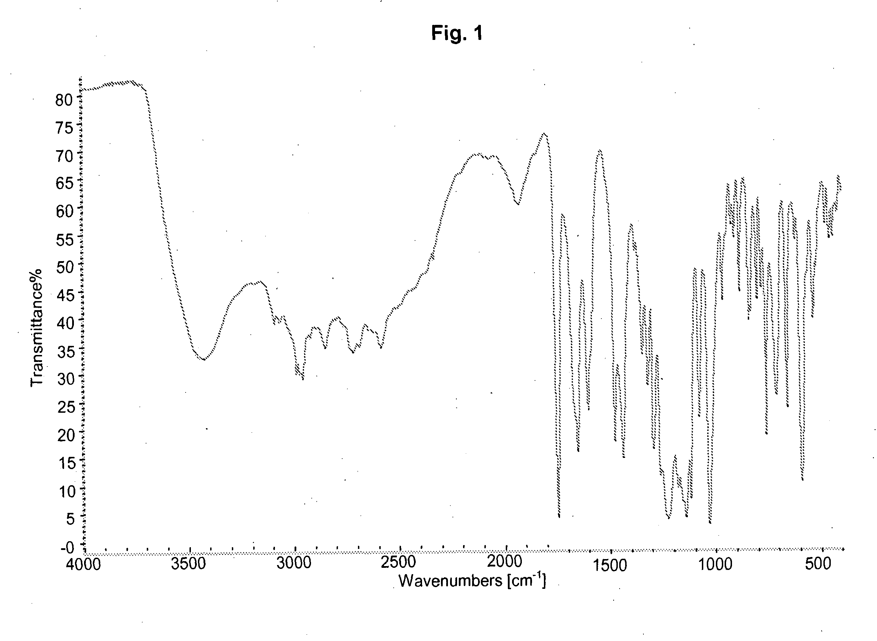

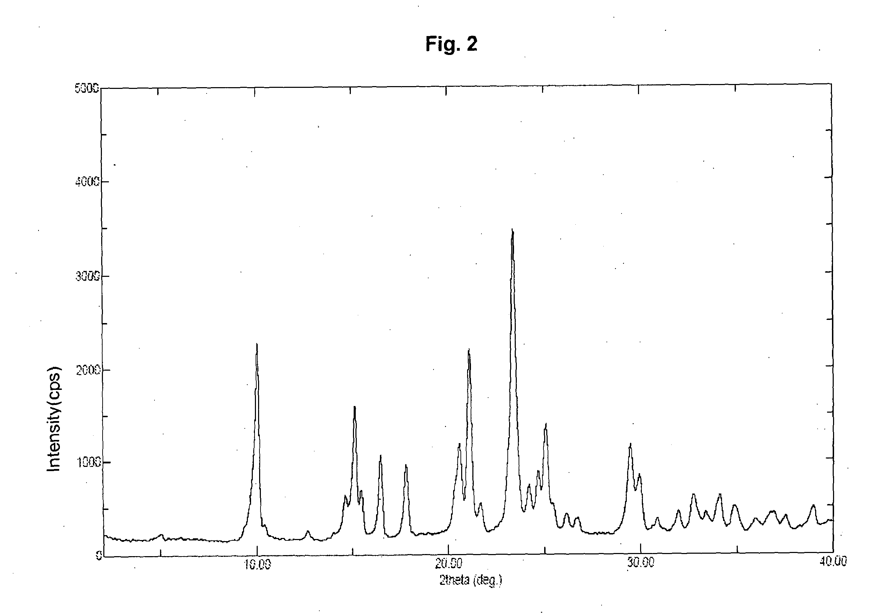

Crystalline Form I of clopidogrel 5-sulfosalicylate is characterized by the melting point within the range of 145-150°C. The enclosed Figures present FTIR spectrum (Fig.1) and X-Ray powder diffraction pattern (Fig.2) of crystalline Form I. FTIR spectrum of crystalline Form I of clopidogrel 5-sulfosalicylate exhibits characteristic absorption bands at: 3426, 2958, 2852, 2717, 2585, 1748, 1654, 1604, 1478, 1438, 1322, 1295, 1226, 1143, 1119, 1079, 1031 cm⁻¹ (Fig. 1). Crystalline Form I of clopidogrel 5-sulfosalicylate also shows characteristic peaks on a X-ray Powder Diffraction pattern (XRPD) expressed in degrees two-theta at: 10.0, 15.1, 16.5, 17.8, 20.6, 21.1, 23.4, 25.1, 29.4. Detailed analysis of XRPD diffraction pattern for Form I of clopidogrel 5-sulfosalicylate is provided in Table 1.

**Table 1. Characteristic peaks, interplanar distances and relative intensities of diffraction lines in the XRPD pattern for crystalline Form I of clopidogrel 5-sulfosalicylate**

| **2θ (±0.2°θ)** | **d (Å)** | **I/Iₒ** |
|---|---|---|
| 10.0 | 8.80 | 65 |
| 14.6 | 6.06 | 12 |
| 15.1 | 5.85 | 42 |
| 16.5 | 5.38 | 28 |
| 17.8 | 4.98 | 25 |
| 20.6 | 4.31 | 27 |
| 21.1 | 4.21 | 60 |
| 21.7 | 4.10 | 8 |
| 23.4 | 3.80 | 100 |
| 24.2 | 3.68 | 10 |
| 24.6 | 3.61 | 16 |
| 25.1 | 3.55 | 33 |
| 26.1 | 3.41 | 6 |
| 26.7 | 3.34 | 5 |
| 29.4 | 3.03 | 29 |
| 29.9 | 2.98 | 18 |
| 30.8 | 2.90 | 5 |
| 31.9 | 2.80 | 7 |
| 32.7 | 2.74 | 12 |
| 33.4 | 2.68 | 5 |
| 34.1 | 2.63 | 12 |
| 34.8 | 2.57 | 8 |
| 36.9 | 2.43 | 5 |
| 37.5 | 2.40 | 6 |
| 38.9 | 2.31 | 7 |

The process of preparation of crystalline Form I of clopidogrel 5-sulfosalicylate consists in the reaction of clopidogrel base dissolved in methylene chloride or chloroform with 5-sulfosalicylic acid. Such conditions assure the formation of crystalline form of the claimed salt.

5-Sulfosalicylic acid is preferably used in the form of crystalline dihydrate. The preferred reaction temperature is from ambient temperature to solvent boiling temperature. After it is filtered off from the reaction mixture the obtained salt, crystalline Form I of clopidogrel 5-sulfosalicylate, is dried in a common dryer or a vacuum dryer at the temperature between room temperature (i.e. about 20°C) to 100°C.

Another method of producing of crystalline Form I of clopidogrel 5-sulfosalicylate consists of adding methylene chloride or chloroform to an oil form of clopidogrel 5-sulfosalicylate and stirring the whole till crystalline salt is precipitated.

It was found that drying process of crystalline Form I of clopidogrel 5-sulfosalicylate, even carried out for more than ten hours, not always provides removal of solvent residue to the level accepted for pharmaceutical substances.

Guideline of the International Conference on Harmonization of Technical Requirements for Registration of Pharmaceuticals for Human Use (ICH) concerning the amounts for residual solvents in pharmaceutical products, so called Guideline Q3C, recommends use of less toxic solvents and possibly the most carefully removing solvents residues from pharmaceutical substances. Guideline Q3C classifies solvents according to their toxicity into three classes:
Class 1: solvents to be avoided- known human carcinogens, strongly suspected human carcinogens, and environmental hazards,
Class 2: solvents to be limited- known animal carcinogens or possible causative agents of neurotoxicity or teratogenicity,
Class 3: solvents with low toxic potential.
Solvents for which there is a lack of any information regarding their toxic activity are placed outside of those classes.

Permitted limits of residual solvents in pharmaceutical substances are described as their concentration [ppm] or maximum permitted daily exposure (PDE) [mg or g/day].

In the process of producing of crystalline clopidogrel 5-sulfosalicylate the solvent is a critical parameter. Only the use of methylene chloride or chloroform in this process ensures the isolation of the product in a stable crystalline form. Both methylene chloride and chloroform belong to solvents from Class 2, so their amount in pharmaceutical products is strictly limited. Acceptable limit for methylene chloride is 600 ppm, and for chloroform is 60 ppm. The residual solvents, particularly those with proved toxicity and defined limits, should be carefully removed from pharmaceutical substances to ensure patient safety. It was found that, drying of crystalline Form I of clopidogrel 5-sulfosalicylate to remove the residue of methylene chloride or chloroform to the limits which are acceptable for pharmaceutical substances creates some problems. It is a long-lasting and energy-consuming process so is not economically optimal. It was found that clopidogrel 5-sulfosalicylate obtained from a solution in methylene chloride needs drying for several days in vacuum dryer, since only after that the concentration of methylene chloride achieves acceptable limit of 600 ppm.

The long-lasting drying is not only energy-consuming process but may also be adverse to the stability of dried substance, particularly when drying is carried out at elevated temperatures.

Classical process of crystallization from a solvent other than methylene chloride and chloroform for clopidogrel 5-sulfosalicylate is completely useless. Dissolving of crystals would be an irreversible process and without re-adding of methylene chloride or chloroform the clopidogrel 5-sulfosalicylate would not be isolated in a solid form.

The inventors of the present invention found that suspending of crystalline Form I of clopidogrel 5-sulfosalicylate having higher than acceptable content of methylene chloride or chloroform in a solvent chosen from the group of esters, ketones and ethers results in complete (below the limit of detection) removal of the residues of methylene chloride and chloroform. Moreover it was surprisingly found that such process causes polymorphic transformation of crystals from Form I into a new crystalline form - Form II.

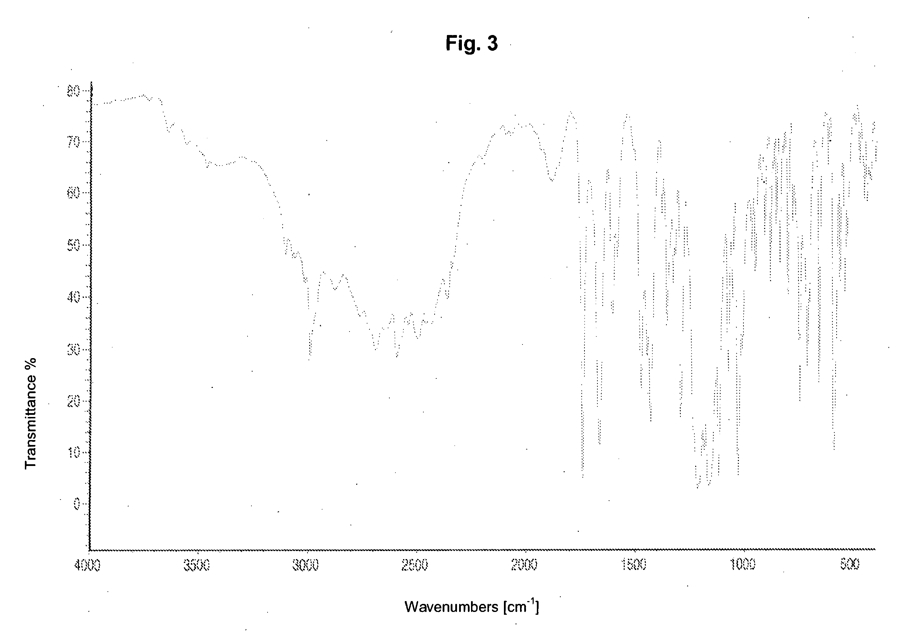

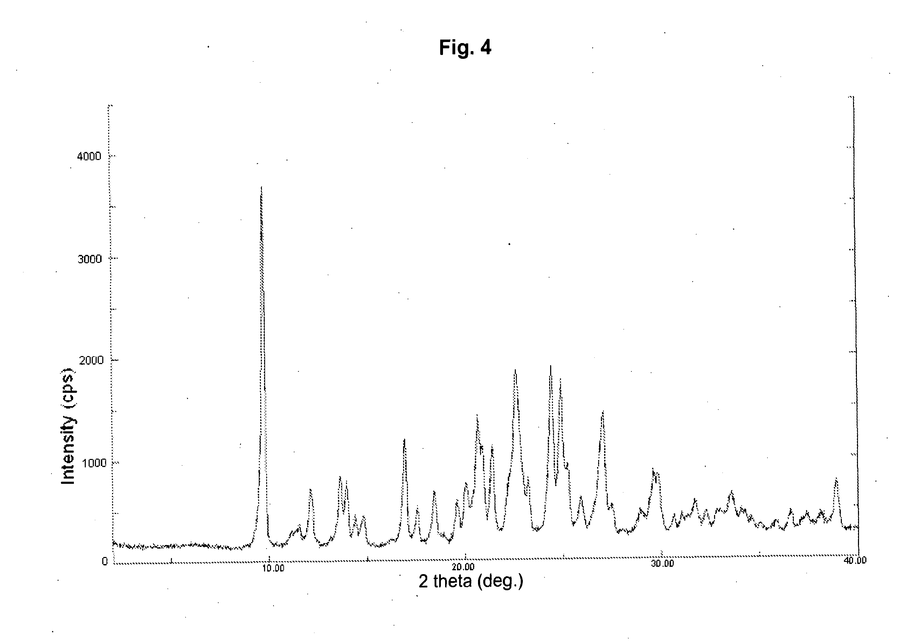

New crystalline Form II of clopidogrel 5-sulfosalicylate is extremely stable, non-hygroscopic and easily undergoes the pharmaceutical processing. This form has the melting point within the range of 178-188°C. The enclosed Figures present FTIR spectrum (Fig.3) and X-Ray powder diffraction pattern (Fig.4) of said crystalline Form II of clopidogrel 5-sulfosalicylate. FTIR spectrum of Form II of clopidogrel 5-sulfosalicylate exhibits characteristic absorption bands at: 3102, 2879, 2593, 1742, 1664, 1585, 1474, 1434, 1359, 1291, 1216, 1164, 1122, 1078, 1032 cm⁻¹. Crystalline Form II of clopidogrel 5-sulfosalicylate also shows characteristic peaks on a X-ray Powder Diffraction pattern (XRPD) in degrees two-theta at: 9.8, 13.6, 14.0, 16.9, 20.1, 20.7, 20.9, 21.4, 22.2, 22.6, 23.2, 24.4, 24.9, 25.3, 27.1, 29.6, 29.9, 38.9. Detailed analysis of XRPD diffraction pattern is provided in Table 2.

**Table 2. Characteristic peaks, interplanar distances and relative intensities of diffraction lines in the XRPD pattern for crystalline Form II of clopidogrel 5-sulfosalicylate**

| **2θ (±0.2°θ)** | **d (Å)** | **I/Iₒ** |
|---|---|---|
| 9.8 | 9.05 | 100 |
| 11.5 | 7.66 | 10 |
| 12.1 | 7.28 | 20 |
| 13.6 | 6.49 | 23 |
| 14.0 | 6.34 | 22 |
| 14.4 | 6.15 | 11 |
| 14.8 | 5.97 | 13 |
| 16.9 | 5.23 | 33 |
| 17.6 | 5.05 | 15 |
| 18.4 | 4.81 | 19 |
| 19.6 | 4.53 | 16 |
| 20.1 | 4.42 | 21 |
| 20.7 | 4.29 | 40 |
| 20.9 | 4.24 | 31 |
| 21.4 | 4.14 | 29 |
| 22.2 | 4.00 | 23 |
| 22.6 | 3.93 | 51 |
| 23.2 | 3.83 | 23 |
| 24.4 | 3.64 | 53 |
| 24.9 | 3.57 | 49 |
| 25.3 | 3.52 | 26 |
| 25.9 | 3.43 | 16 |
| 27.1 | 3.29 | 40 |
| 27.5 | 3.24 | 15 |
| 29.6 | 3.01 | 23 |
| 29.9 | 2.99 | 22 |
| 31.7 | 2.82 | 16 |
| 32.2 | 2.77 | 13 |
| 32.8 | 2.72 | 13 |
| 33.0 | 2.71 | 13 |
| 33.5 | 2.67 | 17 |
| 33.6 | 2.66 | 18 |
| 33.7 | 2.65 | 17 |
| 34.1 | 2.63 | 14 |
| 34.2 | 2.62 | 13 |
| 38.1 | 2.36 | 13 |
| 38.9 | 2.31 | 21 |

Process for preparation of crystalline Form II of clopidogrel 5-sulfosalicylate, consists in that crystalline Form I of clopidogrel 5-sulfosalicylate is suspended in a solvent selected from the group of esters, ethers and ketones or a mixture thereof, said suspension is stirred, optionally at elevated temperature, and then crystalline Form II of clopidogrel 5-sulfosalicylate is isolated and dried. Preferably said suspension is kept between ambient temperature and the solvent boiling temperature. The solvent boiling temperature is particularly preferred. The solvent used is selected from the group of esters, ethers and ketones or a mixture thereof, preferably from esters and the most preferably it is selected from solvents from Class 3 or solvents outside of toxicity classes, such as, for example, ethyl acetate, butyl acetate, ethyl formate, isopropyl acetate, isobutyl acetate, methyl acetate, propyl acetate, diethyl ether, tert-butyl methyl ether, tetrahydrofuran, isopropyl ether, acetone, methyl ethyl ketone, methyl isobutyl ketone, methyl isopropyl ketone,. The used solvent should sparingly dissolve clopidogrel 5-sulfosalicylate and should be miscible with methylene chloride and chloroform. Ethyl acetate is the most preferred solvent.

For producing crystalline Form II of clopidogrel 5-sulfosalicylate said salt in crystalline Form I is suspended in the chosen solvent and the suspension is stirred at the temperature from the ambient temperature, i.e. about 20°C, to the solvent boiling temperature. The time of suspending depends on the selected solvent, used temperature and stirring effectiveness. Then the suspension is chilled (if it was heated) to the room temperature. The precipitate is filtered off on vacuum filter or centrifuged, optionally it is rinsed with a small amount of solvent, which was used for suspension preparation, and it is dried. The best results were achieved with solvents selected from esters, particularly with ethyl acetate. The obtained product, crystalline Form II of clopidogrel 5-sulfosalicylate is characterized by very high chemical purity and assay, and moreover it is free of residues of methylene chloride or chloroform (the analysis has shown that the content of those solvents is below the detection limit). The presence of residual solvent used for preparing the suspension was detected but in amounts much below acceptable limits. Taking into consideration fact that solvents for preparing the suspension are selected from solvents with low potential toxicity, their presence in the product in amounts 300-600 ppm does not cause a real danger, when acceptable content of solvents from Class 3 is 5.000 ppm.

Similarly to crystalline Form I, also crystalline Form II of clopidogrel 5-sulfosalicylate exhibits better stability in elevated moisture in comparison with clopidogrel hydrogen sulfate. Clopidogrel hydrogen sulfate undergoes hydrolysis and racemization in a substantial degree. The racemization process is of great importance due to known toxicity of clopidogrel laevorotatory isomer.

The present invention provides also pharmaceutical compositions comprising clopidogrel 5-sulfosalicylate as described above and pharmaceutically acceptable carrier or excipient.

New clopidogrel salt with 5-sulfosalicylic acid may be formulated in a pharmaceutical composition according to known conventional methods. For oral administration the pharmaceutical composition may be in a dosage form of, for example, tablets, capsules, powders, solutions, syrups or suspensions prepared with conventional carriers and excipients. Tablet is a preferred oral unit dosage form containing clopidogrel salt with 5-sulfosalicylic acid.

The invention further provides new clopidogrel salt with 5-sulfosalicylic acid for use in medicine.

In another aspect, the present invention provides the use of clopidogrel salt with 5-sulfosalicylic acid as a platelet aggregation inhibitor for the manufacture of a medicament for treatment of platelet aggregation related diseases.

The following Examples illustrate the invention, and should not be read as limiting the scope of invention in any way.

### Reagents and Instrumentation:

FTIR spectra were recorded with Thermo Nicolet spectrometer from Spectro Lab in the spectrum range 4000-400 cm⁻¹. The samples were prepared in the form of a KBr tablet. Obtained results are depicted in Fig.1 and Fig.3.

XRPD analyses were conducted by means of a MiniFlex X-ray diffractometer from Rigaku, with Cu Kα radiation, filter Kβ. Lamps parameters were 30 kV and 15 mA. Divergence and scattering slits were set at 4,2°, and a receiving slit was set at 0,3 mm. Deflection of rays was detected with scintillation detector Nal. The θ/2θ continuous scan at 1°/min with a step of 0,02° from 2,0 to 40° 2θ was used. The samples were prepared by pressing the preparation in quartz holders. Obtained results are depicted in Fig.2 iFig.4.

Chemical purity was determined by HPLC: Alliance 2695 chromatograph from Waters, detection UV, column C18 150×4.6 mm.

Enantiomeric purity was determined by HPLC: Alliance 2695 chromatograph from Waters, detection UV, chiral column 150x4.0 mm.

Content of residual solvents was determined by gas chromatography with headspace and flame ionization detector (FID): chromatograph GC 3800 from Varian, headspace: Teledyne Tekmar.

Clopidogrel camphorsulfonate used as starting material is commercially available.

### Preparation of clopidogrel base

Clopidogrel camphorsulfonate 34 g (0,062 mol) was placed in glass beaker and 500 ml of water and 500 ml of methylene chloride were added. Potassium carbonate was added with stirring until the release of carbon dioxide ceases. The reaction mixture was vigorously stirred for 30 minutes, and the mixture was separated. The organic layer was dried with anhydrous magnesium sulfate, and evaporated to dryness. 20 g of clopidogrel base as pale-yellow liquid was obtained.

### Example 1

Clopidogrel (base) 16 g was dissolved in 100 ml methanol and 12.7 g (0.05 mol) of 5-sulphosalicylic acid dihydrate was added with stirring. The whole was stirred at the temperature of 30°C for 30 minutes, and then the solution was evaporated to dryness under reduced pressure to obtain clopidogrel 5-sulfosalicylate as pale yellow oil (quantitative yield).
Chemical purity by HPLC: 99.43 %.
Enantiomeric purity by HPLC: 99.53 %.

### Example 2

30 ml of methylene chloride was added to 10 g of clopidogrel 5-sulfosalicylate in an oil form. The whole was stirred under reflux for 30 minutes, and subsequently was cooled to the room temperature. The white precipitate was filtered off - crystalline Form I of clopidogrel 5-sulfosalicylate (quantitative yield).
Chemical purity by HPLC: 99.58 %.
Enantiomeric purity by HPLC: 100 %.

### Example 3

Clopidogrel base (20 g) was dissolved in methylene chloride (100 ml) and 13 g (0.06 mol) of 5-sulfosalicylic acid was added with stirring. The reaction mixture was refluxed for 1 hour. The mixture was then cooled to room temperature and white solid product was filtered off - clopidogrel 5-sulphosalicylate in a crystalline Form I (quantitative yield).
Chemical purity by HPLC: 99.53 %.
Enantiomeric purity by HPLC: 100 %.

### Example 4

Clopidogrel base (500 g) (1.557 mol) was dissolved in methylene chloride (5 liters) and 395 g (1.557 mol) of 5-sulfosalicylic acid dihydrate was added with stirring. Almost immediately precipitation is occured. The mixture was stirred at room temperature for 30 minutes, and then white solid product was filtered off - clopidogrel 5-sulfosalicylate in a crystalline Form I (quantitative yield).
Chemical purity of HPLC: 99.49 %.
Enantiomeric purity of HPLC: 100 %.

### Example 5

Crystalline Form I of clopidogrel 5-sulfosalicylate (1 kg) containing 2000 ppm of methylene chloride was placed in a 20-liter reactor, equipped with stirrer and reflux condenser. Ethyl acetate (13 liters) was added, then the stirrer was run and the mixture was heated till boiling. The reaction mixture was refluxed for 6 hours, and then it was cooled to room temperature. Afterwards the precipitate was filtered off on pressure filter and washed with ethyl acetate. Obtained crystals were dried in a dryer at the temperature 70°C for 10 hours, 950 g of crystalline Form II of clopidogrel 5-sulfosalicylate were obtained.
Chemical purity by HPLC: 99.62 %.
Enantiomeric purity by HPLC: 100 %.

### Residual solvents:

- methylene chloride: below the limit of detection,
- ethyl acetate: 353 ppm.

### Example 6

Crystalline Form I of clopidogrel 5-sulfosalicylate (24 kg) containing 480 ppm of methylene chloride was placed in a 500-liter reactor, equipped with stirrer and reflux condenser. Ethyl acetate (300 liters) was added, then the stirrer was run and the mixture was heated till boiling. The reaction mixture was refluxed for 6 hours, and then it was cooled to room temperature. After that the precipitate was centrifuged and washed with ethyl acetate. Obtained crystals were dried in vacuum dryer. 21 kg of crystalline Form II of clopidogrel 5-sulfosalicylate were obtained.
Chemical purity by HPLC: 99.81 %.
Enantiomeric purity by HPLC: 100 %.

### Residual solvents:

- methylene chloride: below the limit of detection,
- ethyl acetate: 335 ppm.

### Example 7

2.2 g of crystalline Form I of clopidogrel 5-sulfosalicylate and 50 ml of tert-butyl methyl ether were placed in a round bottom flask equipped with stirrer. The suspension was stirred for 24 hours at the ambient temperature (20°C), then the precipitate was filtered off and dried in vacuum dryer at the temperature 45°C under 1,33 kPa (10 Tr). 2 g of crystalline Form II of clopidogrel 5-sulfosalicylate was obtained.
Chemical purity by HPLC: 99.60 %.
Enantiomeric purity by HPLC: 100 %.

### Residual solvents:

- methylene chloride: below the limit of detection,
- tert-butyl methyl ether: 450 ppm.

### Example 8

1 g of crystalline Form I of clopidogrel 5-sulfosalicylate and 10 ml of methyl isopropyl ketone were placed in a round bottom flask equipped with stirrer. The suspension was stirred for 6 hours at reflux, and then it was cooled to the ambient temperature. After that the precipitate was filtered off and dried in vacuum dryer 45°C/1,33 kPa (10 Tr). 0.9 g of crystalline Form II of clopidogrel 5-sulfosalicylate was obtained.
Chemical purity by HPLC: 99.70 %.
Enantiomeric purity by HPLC: 100 %.

### Residual solvents:

- methylene chloride: below the limit of detection,
- methyl isopropyl ketone: 530 ppm.

### Example 9

Moisture influence on clopidogrel hydrogen sulfate and on crystalline Forms I and II of clopidogrel 5-sulfosalicylate has been studied.

Clopidogrel hydrogen sulfate, which was used for comparison, was prepared according to the method described in the patent publication EP-281459, Example 1d.

In this experiment clopidogrel 5-sulfosalicylate crystalline Form I prepared according to Example 3 and crystalline Form II prepared according to Example 5 were used.

Conditions: steam saturation at the temperature of 50°C for 24 hours.

| Clopidogrel salt | Total impurities [%] | | Clopidogrel (-) optical isomer [%] | |
|---|---|---|---|---|
| | START | 24 h | START | 24 h |
| Clopidogrel hydrogen sulfate | 0.25 | 16.13 | 0.07 | 5.97 |
| Clopidogrel 5-sulfosalicylate, crystalline Form I | 0.26 | 0.57 | 0.06 | 0.11 |
| Clopidogrel 5-sulfosalicylate, crystalline Form II | 0.41 | 0.44 | 0.09 | 0.08 |

This data shows that clopidogrel 5-sulfosalicylate, both in Form I and Form II, is moisture resistant. Form II was exceptionally stable; practically no change in content of impurities was observed. Form I of the salt is a little less stable; but the level of impurities increases very slowly.

In case of clopidogrel hydrogen sulfate considerable increase of impurities was observed, and most of them constituted clopidogrel hydrolysis product. Moreover during the experiments the amount of toxic optical isomer of clopidogrel increased significantly.

## Claims

1. Clopidogrel 5-sulfosalicylate.

2. Crystalline Form I of clopidogrel 5-sulfosalicylate according to claim 1 having an X-ray powder diffraction pattern (XRPD) with specific peaks at 2θ degrees (±0.2°θ): 10.0, 15.1, 16.5, 17.8, 20.6, 21.1, 23.4, 25.1, 29.4.

3. Crystalline Form II of clopidogrel 5-sulphosalicylate according to claim 1 having an X-ray powder diffraction pattern (XRPD) with specific peaks at 2θ degrees (±0.2°θ): 9.8, 13.6, 14.0, 16.9, 20.1, 20.7, 20.9, 21.4, 22.2, 22.6, 23.2, 24.4, 24.9, 25.3, 27.1, 29.6, 29.9, 38.9.

4. Process for preparation of clopidogrel 5-sulfosalicylate **characterized in that** clopidogrel in the form of a base is reacted with 5-sulfosalicylic acid.

5. Process for preparation according to claim 4, **characterized in that** clopidogrel is used in the form of a solution.

6. Process for preparation according to any claims 4-5, **characterized in that** 5-sulfosalicylic acid is in a crystalline dihydrate form.

7. Process for preparation according to any claims 4-6, **characterized in that** reaction is carried out at a temperature from ambient temperature to the solvent boiling point temperature.

8. Process for preparation of crystalline Form I of clopidogrel 5-sulfosalicylate, **characterized in that** clopidogrel in the form of a base, dissolved in methylene chloride or chloroform, is reacted with 5-sulfosalicylic acid.

9. Process for preparation according to claim 8, **characterized in that** 5-sulfosalicylic acid is in a crystalline dihydrate form.

10. Process for preparation according to any claims 8-9, **characterized in that** reaction is carried out at a temperature from ambient temperature to the solvent boiling point temperature.

11. Process for preparation of crystalline Form I of clopidogrel 5-sulfosalicylate, **characterized in that** methylene chloride or chloroform is added to clopidogrel 5-sulfosalicylate in an oil form, and the whole is stirred till crystalline salt is precipitated.

12. Process for preparation of crystalline Form II of clopidogrel 5-sulfosalicylate, **characterized in that** crystalline Form I of clopidogrel 5-sulfosalicylate is suspended in a solvent selected from the group of esters, ethers and ketones or mixture thereof, and the suspension is stirred, optionally at elevated temperature, and then crystalline Form II of clopidogrel 5-sulfosalicylate is isolated and dried.

13. Process for preparation according to claim 12, **characterized in that** the suspension is kept between ambient temperature and the solvent boiling point temperature, preferably at the solvent boiling point temperature

14. Process for preparation according to any claims 12-13, **characterized in that** solvent is selected from the group of esters, advantageously the solvent is ethyl acetate.

15. A pharmaceutical composition, which comprises clopidogrel 5-sulfosalicylate as claimed in any one of Claims 1 to 3 and pharmaceutically acceptable carrier or excipient.

16. Clopidogrel 5-sulfosalicylate as defined in any one of claim 1 to 3 for use in medicine.

17. Use of clopidogrel 5-sulfosalicylate as defined in any of claims 1 to 3 in the manufacture of a medicament for treatment of platelet aggregation related diseases.

## Patentansprüche

1. Clopidogrel 5-Sulfosalicylat.

2. Kristallform I von Clopidogrel 5-Sulfosalicylat nach Anspruch 1, **dadurch gekennzeichnet, dass** sie charakteristische Peaks auf Röntgen Pulverdiffraktogramm (XRPD), angegeben im Grad 2θ (±0.2°θ) : 10.0, 15.1, 16.5, 17.8, 20.6, 21.1, 23.4, 25.1, 29.4, aufweist.

3. Kristallform II von Clopidogrel 5-Sulfosalicylat nach Anspruch 1, **dadurch gekennzeichnet, dass** sie charakteristische Peaks auf Röntgen Pulverdiffraktogramm (XRPD), angegeben im Grad 2θ **(±0.2°θ) : 9.8, 13.6, 14.0, .16.9,** 20.1 20.7, **20.9,** 21.4, 22.2, 22.6, 23.2, **24.4, 24.9,** 25.3, 27.1, 29.6, **29.9,** 38.9, aufweist.

4. Verfahren zur Herstellung von Clopidogrel 5-Salicylat, **dadurch gekennzeichnet, dass** man die Clopidogrel Base mit 5-Sulfosalicylsaure umsetzt.

5. Verfahren nach Anspruch 4, **dadurch gekennzeichnet, dass** man Clopidogrel als Losung in einem Losungsmittel verwendet.

6. Verfahren nach einem der Ansprüche 4-5, **dadurch gekennzeichnet, dass** man 5-Sulfosalicylsaure in Form des kristallinen Dihydrats gebraucht.

7. Verfahren nach einem der Ansprüche 4-6, **dadurch gekennzeichnet, dass** man die Reaktion bei einer Temperatur im Bereich von der Raumtemperatur bis zum Siedepunkt des Losungsmittels durchführt.

8. Verfahren zur Herstellung der Kristallform I der Clopidogrel 5-Sulfosalicylat, **dadurch gekennzeichnet, dass** man eine Losung der Clopidogrel Base in Methylenchlorid oder in Chloroform mit 5-Sulfosalicylsaure umsetzt.

9. Verfahren nach Anspruch 8, **dadurch gekennzeichnet, dass** man 5-Sulfosalicylsaure in Form des kristallinen Dihydrats gebraucht.

10. Verfahren nach einem der Ansprüche 8-9, **dadurch gekennzeichnet, dass** man die Reaktion bei einer Temperatur im Bereich von der Raumtemperatur bis zum Siedepunkt des Losungsmittels führt.

11. Verfahren zur Herstellung der Kristallform I von Clopidogrel 5-Sulfosalicylat, **dadurch gekennzeichnet, dass** man die ölige Form von Clopidogrel 5-Sulfosalicylat mit Methylenchlorid oder Chloroform behandelt und die Mischung bis zur Umfallung des kristallinen Salzes rührt.

12. Verfahren zur Herstellung der Kristallform II von Clopidogrel 5-Sulfosalicylat, **dadurch gekennzeichnet, dass** man die Kristallform I von Clopidogrel 5-Sulfosalicylat in einem Losungsmittel, gewählt aus einer aus Ester, Ether und Ketone bzw. deren Mischung bestehenden Gruppe, suspendiert, die erhaltene Suspension, gegebenenfalls bei erhöhter Temperatur, rührt und danach die Kristallform II von Clopidogrel 5-Salicylat isoliert and trocknet.

13. Verfahren nach Anspruch 12, **dadurch gekennzeichnet, dass** man die Suspension bei einer Temperatur im Bereich von der Raumtemperatur bis zum Siedepunkt des Losungsmittels hält, vorzugsweise beim Siedepunkt des Losungsmittels.

14. Verfahren nach einem der Ansprüche 12-13, **dadurch gekennzeichnet, dass** man ein Losungsmittel, gewählt aus der Gruppe von Estern, vorzugsweise Ethylacetat, verwendet.

15. Pharmazeutische Komposition, enthaltend Clopidogrel 5-Sulfosalicylat nach einem der Ansprüche 1-3, mit einem pharmazeutisch zulässigen Träger oder Hilfsstoff.

16. Clopidogrel 5-Sulfosalicylat nach einem der Ansprüche 1-3, zur Verwendung als Arzneimittel.

17. Verwendung von Clopidogrel 5-Sulfosalicylat nach einem der Ansprüche 1-3 zur Herstellung von Arzneimitteln zur Behandlung der Blutplättchenkrankheiten.

## Revendications

1. 5-Sulfosalicylate de clopidogrel.

2. La Forme cristalline I de 5-Sulfosalicylate de clopidogrel selon la revendication 1 **se caractérisant par** les pics sur le diffractogramme de poudre (XRPD) exprimés en degrés 2θ (±0.2°θ): 10.0, 15.1, 16.5, 17.8, 20.6, 21.1, 23.4, 25.1, 29.4.

3. La Forme cristalline II de 5-Sulfosalicylate de clopidogrel selon la revendication 1 **se caractérisant par** les pics sur le diffractogramme de poudre (XRPD) exprimés en degrés 2θ (±0.2°θ): 9.8, 13.6, 14.0, .16.9, 20.1 20.7, 20.9, 21.4, 22.2, 22.6, 23.2, 24.4, 24.9, 25.3, 27.1, 29.6, 29.9, 38.9.

4. Le mode de fabrication de 5-Sulfosalicylate de clopidogrel **caractérisé en ce que** le clopidogrel en forme de base est soumis à la réaction avec l'acide 5-sulfosalicylique.

5. Le mode selon la revendication 4 **caractérisé en ce que** le clopidogrel est utilisé sous forme dissoute dans un solvant.

6. Le mode selon l'une des revendications 4 à 5 **caractérisé en ce que** l'acide 5-sulfosalicylique est utilisé sous forme du dihydrate cristallin.

7. Le mode selon l'une des revendications 4 à 6 **caractérisé en ce que** la réaction est effectuée dans la température à partir de la température ambiante jusqu'à la température d'ébullition du solvant.

8. Le mode de fabrication de la forme cristalline I de 5-Sulfosalicylate de clopidogrel **caractérisé en ce que** le clopidogrel sous forme dissoute dans le chlorure de méthylène ou trichlorométhane est soumis à la réaction avec l'acide 5- sulfosalicylique.

9. Le mode selon la revendication 8 **caractérisé en ce que** l'acide 5- sulfosalicylique est utilisé sous forme de dihydrate cristallin.

10. Le mode selon l'une des revendications 8 à 9 **caractérisé en ce que** la réaction est effectuée dans la température à partir de la température ambiante jusqu'à la température d'ébullition du solvant.

11. Le mode de fabrication de la Forme cristalline I de 5-Sulfosalicylate de clopidogrel **caractérisé en ce qu'**on additionne au clopidogrel sous forme oléagineuse le chlorure de méthylène ou le trichlorométhane et on mélange le tout jusqu'à la précipitation du sel cristallin.

12. Le mode de fabrication de la Forme cristalline II de 5-Sulfosalicylate de clopidogrel **caractérisé en ce qu'**on fait suspendre la Forme cristalline I de 5-Sulfosalicylate de clopidogrel dans un solvant choisi du groupe des esters, éther-oxydes ou cétones ou dans leur mélange. On mélange éventuellement la suspension dans la température élevée et ensuite on isole et on sèche la Forme cristalline II de 5-Sulfosalicylate de clopidogrel.

13. Le mode selon la revendication 12 **caractérisé en ce que** la suspension est maintenue entre la température ambiante et celle d'ébullition du solvant, avantageusement dans la température d'ébullition du solvant.

14. Le mode selon l'une des revendications 12 à 13 **caractérisé en ce qu'**on applique le solvant du groupe des esters, avantageusement l'acétate d'éthyle.

15. La composition pharmaceutique comprenant 5-Sulfosalicylate de clopidogrel selon n'importe quelle revendication 1 à 3, avec un médium ou un véhicule pharmaceutiquement admissible.

16. 5-Sulfosalicylate de clopidogrel selon n'importe quelle revendication 1 à 3 à utiliser comme un médicament.

17. L'utilisation de 5-Sulfosalicylate de clopidogrel selon n'importe quelle revendication 1 à 3 pour la fabrication des médicaments pour traiter les maladies liées avec l'agrégation des plaquettes sanguines.
